# EUROPEAN PATENT SPECIFICATION

(11) **EP 0 751 945 B1**
(45) Date of publication and mention of the grant of the patent: **01.07.1998**
(21) Application number: 95910282.3
(22) Date of filing: 16.02.1995
(51) Int. Cl.: C07D 471/04

(54) **PROCESS FOR PREPARING (1H-TETRAZOL-5-yl)TETRAZOLO 1,5-a] QUINOLINES AND NAPHTHYRIDINES**
VERFAHREN ZUR HERSTELLUNG VON (1H-TETRAZOL-5-YL)TETRAZOLO[1,5-A]QUINOLINEN UND NAPHTHRIDINEN
PROCEDE DE PREPARATION DES (1H-TETRAZOL-5-yl)TETRAZOLO 1,5-a] QUINOLEINES ET NAPHTYRIDINES

(30) Priority: 25.03.1994 US 218280
(43) Date of publication of application: 08.01.1997
(73) Proprietor: MERRELL PHARMACEUTICALS INC., Cincinnati, Ohio 45215-6300 (US)
(72) Inventor: OLMSTEAD, Kay, K., Togok-dong 963 (KR)
(74) Representative: Fischer, Hans-Jürgen, Dr.
(86) International application number: US9501967
(87) International publication number: WO9526348

(56) References cited:
- EP-A- 0 177 923
- DE-A- 2 166 398
- US-A- 4 496 569

## Description

### BACKGROUND OF THE INVENTION

The present invention relates to a process for preparing compounds containing two tetrazole rings with one of the tetrazole rings fused into a tricyclic ring system and the second being a substituent on that ring system. Similar compounds described in United States Patent No. 4,496,569 have known utility as antiallergic agents. Particularly, they are useful in the treatment of conditions in which antigen-antibody reactions are responsible for disease, for example, extrinsic asthma, hay fever, urticaris, eczema or atopic dermatitis and upper respiratory conditions such as allergic rhinitis.

It has heretofore been believed that conversion by azide reaction of a nitrile substituent in a multiple ring system into a tetrazole required the presence of an acid promoter, such as ammonium chloride. In quinolic or naphthyridinoic bicyclic ring systems wherein the primary numbered ring further comprises a 1-α-leaving group and also a nitrile substituent, reaction with azide produces a tricyclic tetrazole-fused compound with a tetrazole substitution replacing the nitrile. Typically, alkali or tetraalkyl azides are used as a common source for azide. However, alkali and tetraalkyl azides were not thought to have been a sufficiently reactive form of azide to convert a nitrile substituent in a multiple ring system into the corresponding tetrazole.

Thus, ammonium chloride was believed to have been necessary in order to convert the sodium azide into the more reactive ammonium azide or hydrazoic acid, which then more easily reacts with the nitrile to form a tetrazole. However, as both ammonium azide and hydrazoic acid are toxic and shock sensitive and hydrazoic acid is extremely volatile, it is desirable to avoid formation and/or use of these compounds in a synthetic scheme.

### SUMMARY OF THE INVENTION

The present invention describes a process for the synthesis of a compound of the formula: 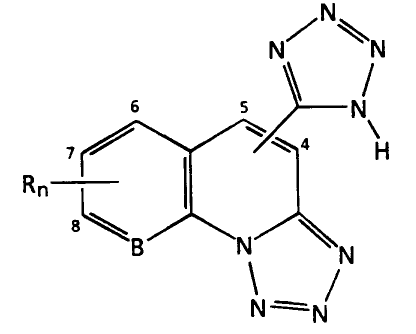 wherein n is 0, 1 or 2; R is C₁₋₄ alkyl, C₁₋₄ alkoxy, halogen, methylmercapto, methylsulfonyl or two R's can be combined as methylenedioxy; B is either nitrogen or CH; with the proviso that, when R is methylmercapto or methylsulfonyl, then n must be 1;
comprising,
reacting in an appropriate solvent,
a nitrile substituted bicyclic compound of the formula: 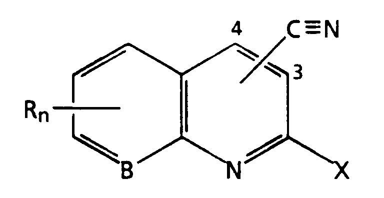 wherein X is a suitable leaving group such as chlorine, fluorine, bromine, iodine or SO₂R¹, wherein: 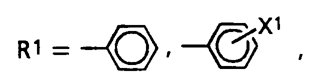 -C₁₋₆ straight chain alkyl, -CF₃, X¹ is H, -CH₃, Br or Cl; and the nitrile is substituted at either the position marked 3 or 4;
with a suitable amount of an appropriate alkali metal azide or tetraalkyl ammonium azide.

### DETAILED DESCRIPTION OF THE INVENTION

As used herein "C₁₋₄ alkyl" means any saturated or branched chain hydrocarbon radical of from 1 to 4 carbon atoms. Included within the scope of this term are methyl, ethyl, n-propyl, isopropyl, n-butyl, isobutyl, t-butyl, and the like.

As used herein "C₁₋₄ alkoxy" means any saturated straight or branched chain radical containing oxygen and from one to 4 carbon atoms, wherein the radical is centered on the oxygen. Included within the scope of this term are methoxy, ethoxy, n-propoxy, isopropoxy, n-butoxy, isobutoxy, t-butoxy and the like.

As used herein "halogen" means fluorine, chlorine or bromine.

The process of the invention proceeds according to the reaction outlined in Scheme A 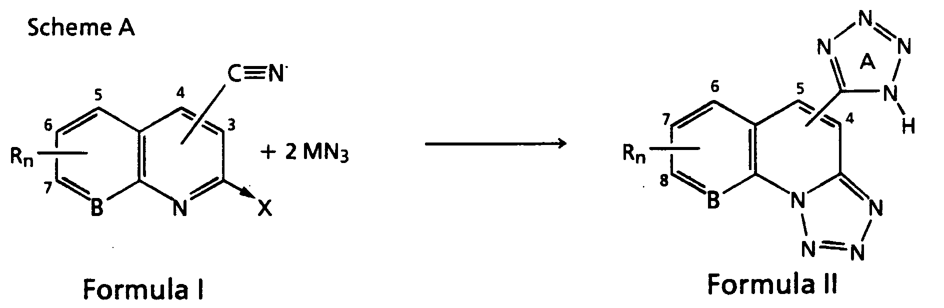 wherein n is 0, 1 or 2, R is C₁₋₄ alkyl, C₁₋₄ alkoxy, lower halogen, methylmercapto, methylsulfonyl, or two R's can be combined as methylenedioxy; B is either nitrogen or CH; and X is a suitable leaving group such as chlorine, fluorine, bromine or SO₂R¹ wherein: 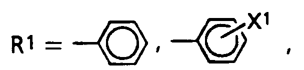 -C₁₋₆ straight chain alkyl, -CF₃, X¹ is H, -CH₃, Br or Cl; M is a lower alkali metal cation or a tetraalkyl ammonium azide; with the proviso that, when R is methylmercapto or methylsulfonyl, then n must be 1.

The starting material in Scheme A (Formula I), a halocyanide, comprises a nitrile substituent at either position 3 or 4 of the bicyclic ring. The nitrile substituent at the 3 or 4 position of the bicyclic starting compound (Formula I) becomes a tetrazole substituent at the 4 or 5 position, respectively, of the fused tricylic system (Formula II) as indicated. The R substituent may be attached at only the 5, 6 or 7 position of Formula I, and appears at the 6, 7 or 8 position, respectively of Formula II, as indicated. For example, the 5 position of Formula I (bicyclic compound) corresponds to the 6 position of Formula II (fused tricyclic system). In a similar manner, positions 6 and 7 correspond to positions 7 and 8 of the formula II. When n is 0, positions 5, 6 and 7 contain an atom of hydrogen. When n is 1 or 2 and R is not either methylmercapto or methyl sulfonyl, R may be present at any of the positions 5, 6 or 7 or combinations thereof. R may be methylmercapto or methylsulfonyl when n is 1. When two R groups are combined to form a methylenedioxy, the substitution occurs at adjacent positions. For example, 5 and 6 or 6 and 7.

The nitrile substituted bicyclic starting material is reacted with a suitable amount of an appropriate alkali metal or tetraalkyl ammonium azide in an appropriate solvent under effective time and temperature conditions until formation of the desired end product is complete.

As used herein "appropriate alkali metal azide" means any inorganic azide comprising the azide anion N₃⁻ and a lower atomic weight alkali metal cation. As example, there may be mentioned lithium azide, sodium azide, potassium azide. The preferred alkali metal azide is sodium azide. As used herein "appropriate tetraalkyl ammonium azide" means an organic azide of the formula "(R₄N)⁺ N₃⁻", wherein R can be a straight or branched lower alkyl of C₁-C₁₈. As examples there may be mentioned tetra-methyl ammonium azide, tetraethyl ammonium azide, tri-methyl-ethyl ammonium azide, dimethyl-diethyl ammonium azide and tetrabutyl ammonium azide.

As used herein, a "suitable amount" an appropriate alkali metal azide can range from about 2.0 to about 5.0 molar equivalents relative to the nitrile substituted bicyclic starting compound. The preferred amount is about 2.1 molar equivalents.

As used herein "appropriate solvent" means a solvating compound suitable for solvating the nitrile and azide reactants in a manner to facilitate formation of a tetrazole. For example, dipolar, aprotic solvents may be employed. As examples, there may be mentioned dimethyl formamide and dimethyl sulfoxide. The preferred dipolar, aprotic solvent is dimethylformamide. Additional solvents which can be used are dimethylacetamide, N-methylpyrrolidinone, tetramethylene sulfone (sulfolane) and the like.

As used herein, "effective time and temperature" means a reaction time and temperature controlled in a manner so as to facilitate the formation of the reaction product. An effective time is a period sufficient for product formation. It can be dependent upon temperature. An effective temperature is one where the reactants have sufficient energy to react within a reasonable time, but not too energetic so as to cause undesired side reactions, or one where the reaction product degrades. For example, an effective reaction time is generally about 1 to about 48 hours, preferably from about 2 to about 24 hours and most preferably from about 4 to about 8 hours. An effective temperature is generally between about 20°C to about 150°C, preferably from about 90°C to about 125°C and most preferred from about 105°C to about 120°C.

From an appropriately substituted acetanilide can be prepared a starting material which is 2-chloro-substituted, where B is CH, and the nitrile is attached at the three position in Formula I (2-chloro-3-cyanoquinoline). The acetanilide can be heated with phosphoryl chloride and dimethylformamide to give the corresponding 2-chloro-3-quinolinecarboxaldehyde. The process is discussed in detail by Meth-Cohn et al., J. Chem. Soc. Perkin Trans. 1, 1981, 1520, which is herein incorporated by reference. The chloroquinoline carboxaldehyde is then reacted with hydroxylamine hydrochloride, formic acid and sodium formate while heating to give the corresponding 3-cyano-2(1H)-quinolinone. This is then heated with an excess of phosphoryl chloride to give the desired 2-chloro-3-cyanoquinoline.

Alternatively, it is possible to obtain the desired 2-chloro-3-cyanoquinoline directly from an appropriate acetanilide. The acetanilide is heated with dimethylformamide and phosphorus oxychloride and, after the initial reaction is complete, hydroxylamine (hydrochloride) is added to the reaction mixture and the product indicated earlier is isolated. Thus, cyclization to a quinoline takes place and a cyano substituted product is obtained.

While all of the basic reactants are the same, the latter procedure for preparing the cyano compound differs from the former one in that the reaction is not carried out step wise with isolation of some type of product after each step of the procedure. With this difference in procedure, the actual series of reactant products involved in the two procedures is not identical. Thus, with acetanilide as the starting material, the reaction with dimethylformamide and phosphoryl chloride actually gives, in solution, the cyclized quinoline with a 3-iminium [-CH=N⁺=] substituent. This iminium (salt) can actually be used as such in solution without resorting to an aqueous workup and isolation wherein the iminium is changed to the corresponding (quinoline)-3-carboxaldehyde. In the step wise procedure, the carboxaldehyde is reacted with hydroxylamine to give the oxime which is then dehydrated to the nitrile but, in the course of this reaction in the quinoline procedure under consideration here, the 2-chloro substituent is hydrolyzed to a ketone and an additional separate step is needed to get back to 2-chloro-substitution. In contrast, in the one-step procedure, the iminium salt can be considered as an aldehyde equivalent and it reacts directly with hydroxylamine to give the oxime. But, since an excess of dehydrating agent is present (phosphoryl chloride), the oxime is immediately dehydrated to the nitrile without affecting the 2-chloro atom. Although the procedure is described above for an aldehyde equivalent (iminium salt), it is possible to carry out the same process on aldehydes too. That is, reaction of an aldehyde with phosphorus oxychloride and hydroxylamine also gives a nitrile directly.

The method above can be generalized to provide a process for the general conversion of an aldehyde or an aldehyde equivalent (such as an iminium salt) to the corresponding nitrile by reaction with hydroxylamine and phosphoryl chloride. The process as described herein can be further generalized to include the immediately preceding step of the formation of an aldehyde or aldehyde equivalent as obtained in the synthesis of the iminium intermediates used in the present application or aldehydes as obtained from an aromatic compound by a Vilsmeier-type reaction.

### Example 1

2-Chloro-3-cyanoquinoline (1.89 g, 0.01 mole) and sodium azide (1.37 g, 0.021 mole) were mixed together at room temperature (15°C to 30°C) in 20-25 ml dimethylformamide in a 100 ml round bottom flask equipped with a stirrer, a condenser, and a thermometer. The headspace of the flask was flushed with nitrogen to a bleach scrubber throughout the reaction. The mixture was gradually heated to a preset temperature between 90°C and 125°C until all the mono-tetrazole intermediate disappeared (confirmed by HPLC). This takes typically 2 to 24 hours. Once the reaction is complete, the reaction is cooled to room temperature (15°C to 30°C) and a 1 mL aliquot of 0.01N NaNO₂ was added. In a well ventilated hood, the mixture was acidified to pH=2 with dilute HCl to convert any unreacted aside to NOₓ and N₂ effluent gases. The resulting solid was filtered and washed with water (25 mL). Yields range typically 90-99% with purity of 98-100% (by weight assay).

### Example 2

2-Chloro-3-cyanoquinoline (30.1 g., 0.159 mol) was added to a suspension of sodium azide (21.75g, 0.335 mol, 1.05 azide equiv.) in 100 mL of dimethylformamide in a 250 mL 3-neck round bottom flask equipped with a stirrer, a condenser, and a thermometer. The mixture was heated to and held at 115°C for 4.5 hours. The reaction progress was measured by liquid chromatography periodically, where the aliquot diluted with equal volume of water has a pH of 10 to 10.5 throughout the reaction. After the reaction was complete, the mixture was cooled to 90°C and half of the solvent was distilled out under vacuum. The mixture was further cooled to room temperature (15°C to 30°C) and 200 mL of water and 20 mL of 1N-NaNO₂ solution was added. The sodium salt of the tetrazole was carefully neutralized with 25 mL of concentrated hydrochloric acid. The resulting suspension was filtered and the wet cake was washed with 300 mL of water. The wet cake was dried to obtain (1H-tetrazol-5-yl)tetrazolo[1,5-a]quinoline.

## Claims

1. A process for the synthesis of a compound of the formula: 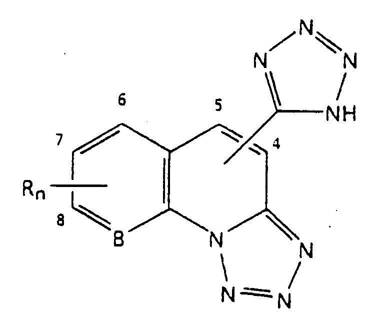 wherein n is 0, 1 or 2; R is C₁₋₄ alkyl, C₁₋₄ alkoxy, halogen, methylmercapto, methylsulfonyl or two R's can be combined as methylenedioxy; B is either nitrogen or CH; with the proviso that, when R is methylmercapto or methylsulfonyl, then n must be 1;
wherein the process comprises the reaction, in a dipolar aprotic solvent, a reaction between:
a) a nitrile substituted bicyclic compound of the formula: 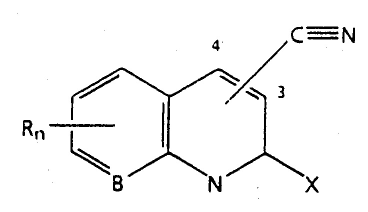 wherein X is a suitable leaving group such as chlorine, fluorine, bromine or SO₂R¹, wherein: 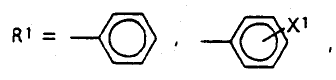 -C₁₋₆ straight chain alkyl, -CF₃, X¹ is H, -CH₃, Br or Cl and the nitrile is substituted at either the 3 or 4 position, with
b) an alkali metal azide or tetraalkyl ammonium azide, characterized by the absence of ammonium chloride or other hydrazoic acid-forming reagent.

2. The process of claim 1 wherein the synthesized compound is:

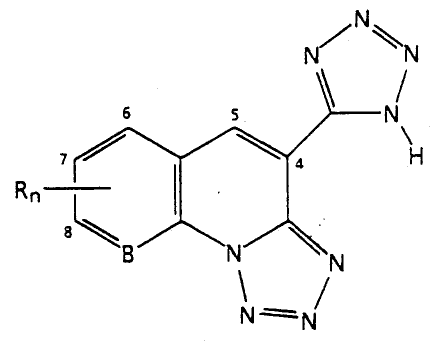

3. The process according to claim 1 wherein the dipolar, aprotic solvent is selected from the group consisting essentially of dimethylformamide, dimethyl sulfoxide, dimethylacetamide, N-methylpyrrolidinone and tetramethylene sulfone.

4. The process according to claim 2 wherein the synthesized compound is 4-(1H-tetrazol-5-yl)tetrazolo[1,5-a]quinoline.

5. The process according to claim 2 wherein the synthesized compound is 7,8-dimethyl-4-(1H-tetrazol-5-yl)tetrazolo[1,5-a]quinoline.

6. The process according to claim 2 wherein the alkali metal azide is selected from the group consisting essentially of lithium azide, sodium azide and potassium azide.

7. The process according to claim 6 wherein the alkali metal azide is sodium azide.

8. The process according to claim 2 wherein the tetraalkyl ammonium azide is selected from the group consisting of tetra-methyl ammonium azide, tetraethyl ammonium azide, tri-methyl-ethyl ammonium azide, tri-ethylmethyl ammonium aside, dimethyl-diethyl ammonium azide and tetrabutyl ammonium azide.

## Patentansprüche

1. Verfahren zur Herstellung einer Verbindung der Formel: 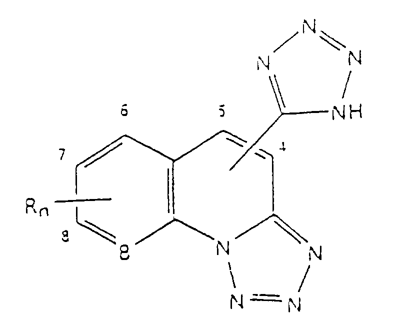 in welcher n gleich 0, 1 oder 2 ist; R für C₁₋₄ Alkyl, C₁₋₄ Alkoxy, Halogen, Methylmercapto, Methylsulfonyl steht oder zwei Substituenten R zu Methylendioxy verbunden sein können; B entweder Stickstoff oder CH ist; unter der Voraussetzung, daß n gleich 1 sein muß, wenn R Methylmercapto oder Methylsulfonyl ist;
wobei das Verfahren die Reaktion in einem dipolaren aprotischen Lösungsmittel umfaßt, und in diese Reaktion eingehen:
a) eine nitrilsubstituierte bizyklische Verbindung der Formel: 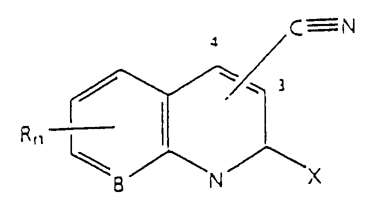 in welcher X eine geeignete Restgruppe wie Chlor, Fluor, Brom oder So₂R¹ ist, mit 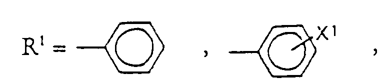 -C₁₋₆ geradkettiges Alkyl, -CF₃, X¹ gleich H, -CH₃, Br oder Cl, und das Nitril in Position 2 oder 3 substituiert ist mit
b) einem Alkalimetallazid oder Tetraalkylammoniumazid, dadurch gekennzeichnet, daß kein Ammoniumchlorid oder ein anderes Stickstoffwasserstoffsäure-bildendes Reagens vorhanden ist.

2. Verfahren gemäß Anspruch 1, wobei die hergestellte Verbindung: 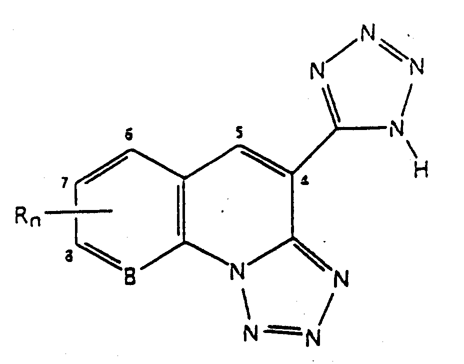 ist.

3. Verfahren gemäß Anspruch 1, wobei das dipolare aprotische Lösungsmittel aus der Gruppe ausgewählt wird, die im wesentlichen Dimethylformamid, Dimethylsulfoxid, Dimethylacetamid, N-Methylpyrrolidinon und Tetramethylensulfon umfaßt.

4. Verfahren gemäß Anspruch 2, wobei die hergestellte Verbindung 4-(1H-tetrazol-5-yl)tetrazol[1,5-a]chinolin ist.

5. Verfahren gemäß Anspruch 2, wobei die hergestellte Verbindung 7,8-Dimethyl-4-(1H-tetrazol-5-yl)tetrazol[1,5-a]chinolin ist.

6. Verfahren gemäß Anspruch 2, wobei das Alklimetallazid aus der Gruppe ausgewählt wird, die im wesentlichen aus Lithiumazid, Natriumazid und Kaliumazid umfaßt.

7. Verfahren gemäß Anspruch 6, wobei das Alkalimetallazid Natriumazid ist.

8. Verfahren gemäß Anspruch 2, wobei das Tetraalkylammoniumazid aus der Gruppe ausgewählt wird, die Tetramethylammoniumazid, Tetraethylammoniumazid, Trimethylethylammoniumazid, Triethylmethylammoniumazid, Dimethyldiethylammoniumazid und Tetrabutylammoniumazid umfaßt.

## Revendications

1. Procédé pour la synthèse d'un composé de formule : 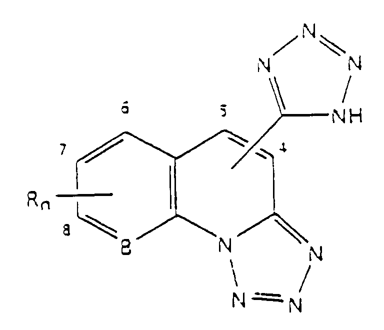 dans laquelle n est 0, 1 ou 2 ; chaque R est un groupe alkyle en C₁-C₄, alcoxy en C₁-C₄, halogéno, méthylmercapto, méthylsulfonyle, ou bien deux R peuvent être combinés en groupe méthylènedioxy ; B est un atome d'azote ou un groupe CH ; à condition que si R est un groupe méthylmercapto ou méthylsulfonyle, alors n soit 1 ;
le procédé comprenant la réaction, dans un solvant aprotique dipolaire, entre
a) un composé bicyclique à substituant nitrile de formule : 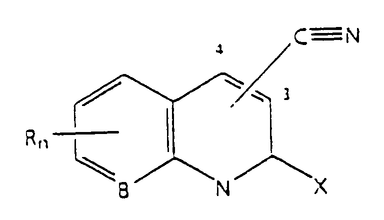 dans laquelle X est un groupe partant approprié tel que le chlore, le fluor, le brome ou SO₂R¹ où R¹ est 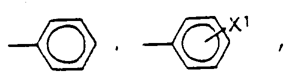 un groupe alkyle à chaîne droite en C₁-C₆ ou -CF₃, X¹ est H, -CH₃, Br ou Cl et le substituant nitrile est fixé à la position 3 ou 4,
et
b) un azoture de métal alcalin ou un azoture de tétraalkylammonium,
caractérisé par l'absence de chlorure d'ammonium ou autre réactif générateur d'acide azothydrique.

2. Procédé de la revendication 1, dans lequel le composé synthétisé est :

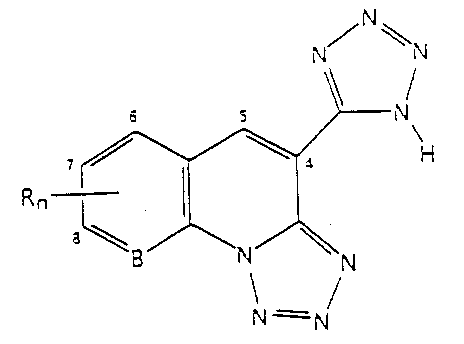

3. Procédé selon la revendication 1, dans lequel le solvant aprotique dipolaire est choisi dans le groupe formé essentiellement par le diméthylformamide, le diméthylsulfoxyde, le diméthylacétamide, la N-méthylpyrrolidinone et la tétraméthylènesulfone.

4. Procédé selon la revendication 2, dans lequel le composé synthétisé est la 4-(1H-tétrazole-5-yl)tétrazolo[1,5-a]quinoléine.

5. Procédé selon la revendication 2, dans lequel le composé synthétisé est la 7,8-diméthyl-4-(1H-tétrazole-5-yl)tétrazolo[1,5-a]quinoléine.

6. Procédé selon la revendication 2, dans lequel l'azoture de métal alcalin est choisi dans le groupe formé essentiellement par l'azoture de lithium, l'azoture de sodium et l'azoture de potassium.

7. Procédé selon la revendication 6, dans lequel l'azoture de métal alcalin est l'azoture de sodium.

8. Procédé selon la revendication 2, dans lequel l'azoture de tétraalkylammonium est choisi dans le groupe formé par l'azote de tétraméthylammonium, l'azoture de tétraéthylammonium, l'azoture de triméthyléthylammonium, l'azoture de triéthylméthylammonium, l'azoture de diméthyldiéthylammonium et l'azoture de tétrabutylammonium.
